(19) Europäisches Patentamt
European Patent Office
Office européen des brevets

(11) Publication number: **0 108 890**
**B1**

(12) **EUROPEAN PATENT SPECIFICATION**

(45) Date of publication of patent specification: **07.12.88**

(51) Int. Cl.[4]: **C 07 D 475/04,** A 61 K 31/495 // C07D475/08, C07D241/26

(21) Application number: **83109291.1**

(22) Date of filing: **19.09.83**

(54) **Pharmaceutically active pteridine derivatives.**

(30) Priority: **20.09.82 GB 8226688**
**12.07.83 GB 8318833**

(43) Date of publication of application:
**23.05.84 Bulletin 84/21**

(45) Publication of the grant of the patent:
**07.12.88 Bulletin 88/49**

(84) Designated Contracting States:
**BE CH DE FR GB IT LI NL SE**

(56) References cited:
**EP-A-0 079 574**
**GB-A-1 293 541**
**GB-A-1 405 246**

(73) Proprietor: **THE WELLCOME FOUNDATION LIMITED**
**183-193 Euston Road**
**London NW1 2BP (GB)**

(72) Inventor: **Nichol, Charles Adam**
**1508 Ward Street**
**Durham North Carolina 27707 (US)**
Inventor: **Reinhard Jr., John Frederick**
**5414 Loyal Place**
**Durham North Carolina 27713 (US)**
Inventor: **Smith, Cary Keith**
**3113 New Hope Church Road**
**Raleigh North Carolina 27604 (US)**
Inventor: **Bigham, Eric Cleveland**
**2511 Foxwood Drive**
**Chapel Hill North Carolina 27514 (US)**

(74) Representative: **Schwabe, Hans-Georg, Dipl.-Ing. et al**
**Patentanwälte Schwabe, Sandmair, Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 108 890 B1

**Description**

The present invention relates to a series of pteridines known as pterins which are analogs of tetrahydrobiopterin and to pharmaceutical formulations containing them. More specifically the invention relates to certain biopterin analogs which are useful in the treatment of Parkinsonism (and other diseases caused by a deficiency of catecholamines in the brain and the peripheral nervous system) and the tetrahydrobiopterin-deficient phenylketonurias.

UK Patent No 1405 246 discloses *inter alia* that pteridines of the formula (O):

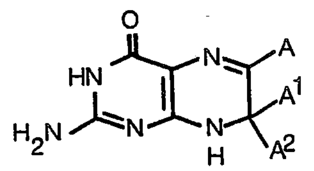

(O)

wherein A is a $C_{1-4}$ alkyl group optionally substituted by a hydroxy group or a halogen atom; and $A^1$ and $A^2$ are the same or different and each is a $C_{1-4}$ alkyl group. These compounds are inhibitors of the enzyme hydroxymethyldihydropteridine pyrophosphokinase and thereby inhibit the growth of microorganisms and, in addition, potentiate the anti-microbial activity of a competitor of p-aminobenzoic acid and/or an inhibitor of dihydrofolate reductase. No CNS activity of these compounds is disclosed. It has now been found that the corresponding tetrahydro pteridines are usedul in the treatment of neurological disorders.

Accordingly, the present invention provides the first medical use of pterin compounds of the formula (I):

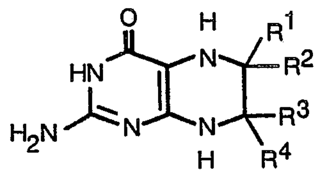

(I)

or a pharmaceutically acceptable salt thereof; wherein $R^1$ is hydrogen, $C_{1-4}$ alkyl, $C_{2-4}$ alkenyl, a group —BX($R^5$)n, or —BZ BX—($R^5$)n wherein Z and X (when n = 1) are the same or different and each is —O—, —$S(O)_q$— or —$NR^6$— wherein q is 0, 1 or 2 and $R^6$ is hydrogen or $C_{1-4}$ alkyl or when n = 0, X is halogen; $R^5$ is hydrogen, up to $C_{12}$ aralkyl, up to $C_{10}$ alkyl or cycloalkyl; B is $C_{1-5}$ alkylene $R^2$ is hydrogen $C_{1-4}$ alkyl, $C_{2-4}$ alkenyl or a group $BOR^7$ wherein B is as hereinbefore defined and $R^7$ is hydrogen or $C_{1-5}$ alkyl; or $R^1$ and $R^2$ together with the carbon atom in the pteridine ring structure to which they are attached, form a $C_{3-7}$ spirocycloalkyl ring; $R^2$ and $R^4$ are hydrogen or methyl; or $R^2$ and $R^3$ together with the carbon atoms in the pteridine ring structure to which they are attached form a $C_{5-7}$ cycloalkyl ring; or a bioprecursor thereof according to formula (IV) or (V)

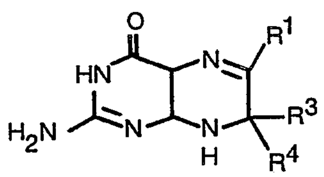

(IV)

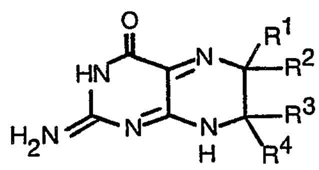

(V)

wherein $R^1$, $R^2$, $R^3$, $R^4$ are as hereinbefore defined with the proviso that when $R^1$ in formula (IV) is methyl, hydroxymethyl or bromomethyl, then $R^3$ and $R^4$ are not both methyl and the compounds 2-amino-4-hydroxy-6-hydroxymethyl-7,7-dimethyl-7,8-dihydropteridine; 2-amino-4-hydroxy-6-methyl-7,7-dimethyl-7,8-dihydropteridine; 2-amino-4-hydroxy-6-bromomethyl-7,7-dimethyl-7,8-dihydropteridine being excluded.

When used herein and where appropriate, the terms alkyl, alkenyl and alkylene cover straight and branched chains.

A preferred group of compounds of the formula (I) is that of formula (II):

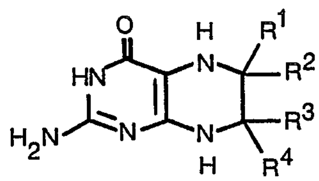

(II)

or a pharmaceutically acceptable salt or bioprecursor thereof as defined by formula (IV) and (V) above; wherein $R^1$ to $R^4$ are as hereinbefore defined and at least one of $R^1$, $R^2$, $R^3$, $R^4$ represents gem disubstitution.

A further preferred group of compounds of the formula (I) is that of the formula (III):

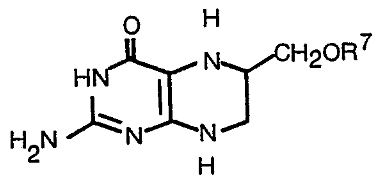

(III)

or a pharmaceutically acceptable salt or bioprecursor thereof as defined by formula (IV) or (V), above wherein $R^7$ is as hereinbefore defined for formula (I).

In a further aspect, the present invention provides novel pterin compounds of the formula (I) or pharmaceutically acceptable salts thereof, that is to say the compounds of the formula (I) as hereinbefore defined with the additional proviso that when both $R^3$ and $R^4$ are methyl, neither $R^1$ nor $R^2$ are methyl and the compounds 2-amino-4-hydroxy-6-hydroxymethyl-7,7-dimethyl-7,8-dihydropteridine; 2-amino-4-hydroxy-6-methyl-7,7-dimethyl-7,8-dihydropteridine; 2-amino-4-hydroxy-6-bromomethyl-7,7-dimethyl-7,8-dihydropteridine being excluded.

A preferred group of novel compounds of the formula (I) is that of the formula (II) or a pharmaceutically acceptable salt thereof; wherein formula (II) is as hereinbefore defined with the additional proviso that when $R^3$ and $R^4$ are methyl, neither $R^1$ nor $R^2$ are methyl.

A further preferred group of novel compounds of the formula (I) are compounds of the formula (III) as hereinbefore defined or bioprecursor thereof as defined by formula (IV) and (V) above.

Suitably $R^1$ is hydrogen, $C_{1-4}$ alkyl or a group $—BX(R^5)n$ as hereinbefore defined. Suitably B is $—CH_2—$, X is oxygen and $(R^5)$, represents hydrogen or a $C_{1-10}$ alkyl group, preferably hydrogen a $C_{1-6}$ alkyl group. Most suitably $R^1$ is hydrogen, methyl, ethyl or a group $—CH_2O(R^5)n$ wherein $R^5$ represents hydrogen or a $C_{1-6}$ alkyl group, such as a methyl or butyl group. Preferably $R^1$ is a group $—CH_2O(R^5)n$ wherein $R^5$ is hydrogen or a $C_{1-6}$ alkyl group.

Suitably, $R^2$ is a hydrogen atom or a $C_{1-6}$ alkyl group. Preferably $R^2$ is a hydrogen atom.

Suitably, at least one of $R^3$ and $R^4$ is methyl.

The compounds of the formula (I) may be formed enzymatically from compounds of the formula (IV) or (V):

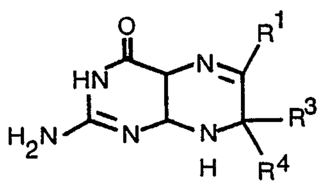

(IV)

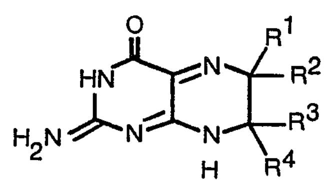

(V)

wherein $R^1$ to $R^4$ are as hereinbefore defined, by the action of dihydrofolate reductase and dihydropteridine reductase respectively. As these enzymes are present in mammals, the compounds of the formulae (IV) and (V) are bioprecursors of the compounds of the formula (I) and the term bioprecursor when used herein relates to these compounds.

Preferred compounds of the formula (I) include (+−)-2-amino-5,6,7,8-tetrahydro-6-methoxymethyl-4(3H)-pteridinone and (+−)-2-amino-5,6,7,8-tetrahydro-6-n-butoxymethyl-4(3H)-pteridinone or pharmaceutically acceptable salts thereof.

The compounds of the formula (I) contain asymetric carbon atoms and the compounds may therefore be obtained in different isomeric forms and as mixtures of isomeric forms. The present invention includes within its scope the different isomeric forms and mixtures of the forms.

It is generally accepted that certain substances known as neurotransmitters are required at the microscopic regions, known as synapses, between nerve cells (neurons) to transmit the nerve impulses throughout the body.

Over thirty substances are known or suspected to be neurotransmitters and each has a characteristic excitatory or inhibitory effect on neurons. Excesses or deficiencies of these transmitters can be manifested as moderate to severe neurological or mental disorders. Whereas neurons are present throughout the body, imbalances of neurotransmitters at synapses of the neurons in the brain are by far the most critical and produce the most pronounced effects.

Of the numerous neurotransmitters known or thought to be operating at synapses, a smaller group collectively known as biogenic amines have received the most study. Particularly important members of this group are the catecholamines such as dopamine and norepinephrine (noradrenaline) and the indole amine, serotonin.

Tetrahydrobiopterin ($BH_4$) is an essential cofactor for the rate limiting enzymes of biogenic amine synthesis, tyrosine and tryptophan hydroxylases, and for the liver enzyme which converts phenylalanine to tyrosine (Kaufman, S. and Fisher, D. B., *Molecular Mechanism of Oxygen activation,* Hayaishi, O. Ed., Acad. Press, N.Y. (1974)). Knowledge of the chemical patholgy of neurological disorders has expanded tremendously during the last two decades. For example, neurological disorders have been described

whose symptoms can be associated with decreases in the number of catecholamine molecules released at certain synaptic sites. As a category, they may be thought of as 'catecholamine-deficiency disorders'. One example is Parkinson's disease (also known as Parkinsonism), where a deficiency in brain dopamine has been linked with the symptoms of rigidity, tremor and akinesia. Another example is chronic preganglionic autonomic insufficiency known as the Shy-Drager syndrome which is associated with both peripheral sympathetic dysfunction and a degeneration of brain neurons in the basal ganglia. The peripheral sympathetic dysfunction most likely reflects a loss of formation and release of the pressor catecholamine norepinephrine, while the rigidity and akinesia most likely reflect a loss of the capacity to form dopamine in certain brain regions.

In all of these cases the catecholamines, whose levels are diminished, are formed through the action of tyrosine hydroxylate which is rate-limiting for their formation. This enzyme requires tyrosine, oxygen and a reduced pterin cofactor, tetrahydrobiopterin ($BH_4$), for activity. While oxygen and tyrosine are not normally limiting for tyrosine hydroxylase, the levels of $BH_4$ may normally be well below the levels required to saturate this enzyme. In fact, there are reports in the scientific literature indicating the levels of this cofactor are severely diminished in Parkinson's disease and in the Shy-Drager syndrome (Nagatsu, T., Neurochem, Intern., *5,* 27 (1983)). Logically, the rate of dopamine biosynthesis would be increased by reversing this deficit. Administration of $BH_4$ has been shown to nearly double striatal dopamine synthesis, as well as noradrenaline synthesis in peripheral nerves (Nagatsu, *vide supra* also Cloutier, G. and Weiner, N., J. Pharm. Exp. Ther., *186,* 75 (1973)). In fact, $BH_4$ administration has been reported to reduce the symptoms of Parkinson's disease (Narabayashi, H., Kond, T., Nagatsu, T., Sugimoto, T. and Matsuura, S., Proc. Japan Acad., *58,* Ser. B, 283 (1082)). A group of psychiatric disorders known as endogenus depression may also involve reduced neuronal formation of catecholamines and serotonin. In fact, $BH_4$ has recently been shown to reduce the symptoms of depression in several patients (Curtius, H., Muldner, H. and Niederwieser, A., J. Neurol. Transmission, *55,* 301 (1982)). This natural cofactor is, however, expensive and unstable, and it penetrates brain poorly. Previously known synthetic cofactor replacements for $BH_4$ all cause tyrosine hydroxylase (TH) to bind its substrate tyrosine more weakly. Thus, the administration of these previously known synthetic cofactors for the acceleration of dopamine biosynthesis in the treatment of catecholamine deficiency causes TH to become unsaturated with tyrosine.

Compounds of formula (I), quite unexpectedly, have been found not to decrease the binding of tyrosine to tyrosine hydroxylase and to promote dopamine formation by this enzyme at rates equal to that observed with the natural cofactor, $BH_4$. Further, certain compounds of formula I enter the brain more easily than $BH_4$ and accelerate dopamine biosynthesis better than the natural cofactor when administered peripherally (orally). Thus it is believed that the administration of compounds of formula (I) for catecholamine deficiency will accelerate dopamine biosynthesis without co-administration of exogenous tyrosine.

Endogenous (psychotic) depression is thought to involve decreased levels of serotonin and noradrenaline at brain synapses. Certain compounds of the formula (I) (those of the formula (II)), function as cofactors for tyrosine hydroxylase but not tryptophan hydroxylase. These compounds have a selective effect therefore when compared with $BH_4$.

Certain other compounds of the formula (I), the compounds of the formula (III) function as cofactors for tyrosine and tryptophan hydroxylases. Thus, these compounds will reduce the symptoms of endogenous depression by promoting the synthesis of both catecholamines and serotonin in the brain. In addition to actions on the central nervous system, the compounds of formula (III) can replace $BH_4$ in the liver and promote the hydroxylation of phenylalanine.

Indeed, a small percentage of all patients with phenylketonuria suffer from a tetrahydrobiopterin-deficient form of phenylketonuria. This "atypical PKU" has been successfully treated with large quantities of $BH_4$ (Nagatsu, *vide supra*). Since these compounds of formula (III) are more lipophilic and have better bioavailability than $NH_4$, considerably smaller doses could be used to treat this atypical PKU. Thus, it is believed that the compounds of formula (III) have at least 2 benefits. First, they may act as cofactors for hepatic phenylalanine hydroxylase to reduce plasma phenylalanine levels. Second, they may act within the brain as cofactors for tyrosine and tryptophan hydroxylase, to correct the $BH_4$ deficient reductions in cerebral catecholamine and serotonin levels which are also seen in atypical PKU. Paradoxically, large amounts of phenylalanine inhibit phenylalanine hydroxylase with $BH_4$ as cofactor. In contrast, compounds of formula (III) have not shown this substrate inhibition and may be expected to remain effective in the presence of large circulating levels of phenylalanine.

Tetrahydropterins, upon reacting with tyrosine, tryptophan or phenylalanine hydroxylase, give rise to an unstable quininoid intermediate which is reduced back to the tetrahydro form by dihydropteridine reductase (DHPR). Compounds of formula (I) have been found to be recycled by DHPR at rates three times faster than that of the natural cofactor. This feature would extend the useful biological life of these compounds, resulting in a favourable duration of action.

The compounds of formula (I) and their salts may be synthesized by methods known in the art for synthesis of compounds having analogous structures.

The compounds of the formula (I) may be prepared by the reduction of the corresponding compound of the formula (IV) or (VI):

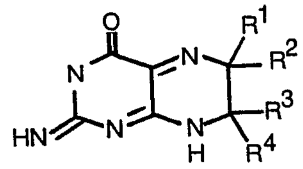

(VI)

This reduction may be carried out enzymatically (particularly in the case of a compound of the formula (IV)) by use of an appropriate enzyme, such as dihydrofolate reductase, for dihydropteridine reductase in the case of a compound of them formula (VI)), or by use of an appropriate reducing agent capable of donating hydrogen, such as hydrogen in the presence of a catalyst, particularly a transition metal catalyst such as platinum oxide or palladium, or a borohydride such as sodium borohydride or sodium cyanoborohydride in an appropriate solvent. Suitable solvents include dilute aqueous acids, such as hydrochloric and trifluoroacetic acids. The reaction is suitably carried out at a non-extreme temperature, i.e. between 0° and 100°C and conveniently at room temperature.

The compounds of the formula (IV) may be prepared by methods well known to those skilled in the art (see for example UK Patents 1303171, 1363064 and 1454165 and *Aust. J. Chem., 34,* 1921—33, scheme 1 illustrates various methods by which compounds of the formula (IV) may be prepared). The compounds of the formula (IV) wherein $R^1$ is a group $—CH_2OR^7$ as hereinbefore defined may be prepared by reduction of the corresponding pteridinone of the formula (VII) (suitably the pteridinone is reduced *in situ* directly to the compound of the formula (I) via the compound of the formula (IV)). The pteridinone (VII):

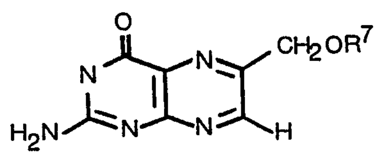

(VII)

may be prepared by hydrolysis of a compound of formula (VIII) with a suitable agent such as an aqueous sodium hydroxide solution.

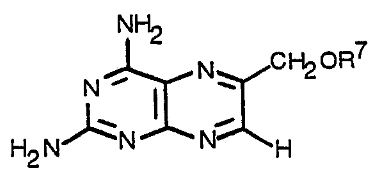

(VIII)

In turn, compounds of formula (VIII) may be prepared by condensation of the compound of formula (IX) with guanidine in the presence of a base, for example sodium alkoxide in an alcohol. A specific example is sodium methoxide in methanol.

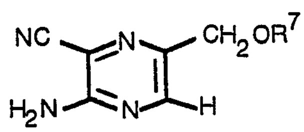

(IX)

Compounds of formula (IX) may be prepared by refluxing a compound of formula (X) in an alcohol $R^7$ OH.

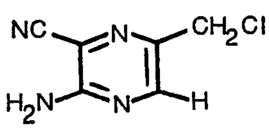

(X)

Compounds of the formula (VI) may be prepared by cyclisation of compounds of the formula (XI).

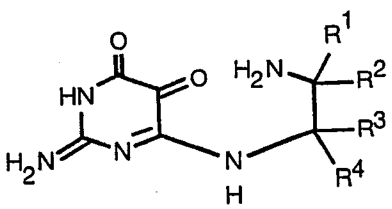

(XI)

The compounds of the formula (XI) may be formed by the oxidation of compounds of the formula (XII):

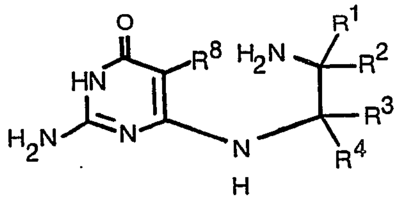

(XII)

wherein $R^1$ to $R^4$ are as hereinbefore defined and $R^8$ is an amino group. The compounds of the formula (VI) are conveniently formed from the compounds of the formula (XII) without the isolation of the compounds of the formula (XI).

The oxidation of the compounds of the formula (XII) wherein $R^8$ is an amino group suitably takes place by the addition of bromine to a solution of a compound of the formula (XII) in methanolic trifluoroacetic acid. Neutralisation, after the reaction is complete, leads to the cyclisation of the compounds of the formula (XI) to give the compounds of the formula (VI). When $R^1$ and $R^2$ are hydrogen, the compounds of the formula (VI) may rearrange to the corresponding compounds of the formula (IV). The compounds of the formula (XII) wherein $R^8$ is an amino group are formed by of the corresponding compounds of the formula (XII) wherein $R^8$ is a nitro group. This reduction may be carried out by catalytic hydrogenation or by dithionite. The compounds of the formula (XII) wherein $R^8$ is a nitro group may be prepared by the condensation of 2-amino-6-chloro-5-nitro-4(3H)-pyrimidinone (formed by the nitration of 2-amino-6-chloro-4(3H)-pyrimidinone) with a compound of the formula (XIII):

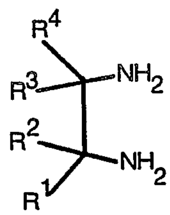

(XIII)

This condensation reaction is suitably carried out at an elevated temperature in an alkanol, such as ethanol.

The compounds of the formula (I) may also be prepared by the reaction of the formula (IV) with a compound $R^2Li$. It may be advisable to prevent anion formation in the compound of the formula (XIV) by reaction with a suitable protecting agent, for example a silylating agent, before the reaction with the compound $R^2Li$. The reaction with $R^2Li$ may conveniently be carried out in an inert aprotic solvent such as an ether, for example diethyl ether, at between −15° and 50°C, for example at room temperature.

It is believed that compounds of this invention may be used to treat Parkinson's disease, endogenous depression, orthostatic hypotension, muscular dystonia and other disorders which arise from deficiencies of catecholamines and serotonin at the pre-synaptic sites of neuronal junctions. It is also believed that these compounds may be used to treat the $BH_4$-deficient phenylketonurias.

The amount of the active compound, i.e a compound of formula (I), required for use in the above disorders will, of course, vary with the route of administration, the condition being treated, and the person undergoing treatment, but is ultimately at the discretion of the physician. However, a suitable dose for treating these disorders is in the range of from 0.5 to 20 mg per kilogram body weight per day preferably from 1 to 10 mg/kg body weight per day, most preferably from 2 to 7 mg/kg body weight per day, a typical preferred dose in 5 mg/kg body weight per day.

The desired dose is preferably presented as between one and four subdoses administered at appropriate intervals throughout the day. Thus where three subdoses are employed each will lie in the range of from 0.17 to 6.7 mg/kg body weight; a typical dose for a human recipient being 1.7 mg/kg body weight.

If desirable, the catecholamine and serotonin precursors 1-tyrosine or 1-tryptophan may be administered concurrently with a compound of formula (I) at the rate of 25 mg/kg to 1000 mg/kg body weight. These amino acids may be given in the same pharmaceutical formulation, e.g., tablet or capsule, as a compound of formula (I) or in a separate pharmaceutical formulation.

While it is possible for the active compound or compounds to be administered alone as the raw chemicals, it is preferable to present the active compound or compounds as pharmaceutical formulations. Formulations of the present invention comprise a compound of formula (I) together with one or more pharmaceutically acceptable carriers thereof and optionally any other active therapeutic ingredients.

The carrier(s) must be pharmaceutically acceptable in the sense of being compatible with the other ingredients of the formulation and not deleterious to the recipient thereof. The carrier may contain a preservative agent such as ascorbic acid.

The formulations include those suitable for oral, rectal or parenteral (including subcutaneous, intramuscular and intravenous) administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a finely divided solid carrier and then, if necessary, shaping the product into the desired formulations or packaging in a suitable container.

Formulations of the present invention suitable for oral administration may be presented as discrete units such as capsules, cachets, tablets or lozenges, each containing a predetermined amount of the active compound(s); as a powder or granules; or a suspension in a non-aqueous liquid such as a syrup, an elixir, and emulsion or a draught. The active compound(s) may also be presented as a bolus or depot formulation.

A tablet may be made by compression or molding, optionally with one or more accessory ingredients. Compressed tablets may be prepared by compressing in a suitable mchine, the active compound being in a free-flowing form such as powder or granule, optionally mixed with a binder, lubricant, inert diluent, surface active or dispersing agent. Molded tablets may be made by molding in a suitable machine, comprising a mixture of the powdered active compound(s) with any suitable carrier.

Formulations for rectal administration may be presented as a suppository with a usual carrier such as cocoa butter.

Formulations suitable for parenteral administration can be made sterile and packed as dry solids in sealed sterile nitrogen-purged containers. At the time of parenteral administration a specified amount of sterile water is added to the drug formulation and the resulting solution is administered to the patient with a minimum of delay since the compounds of formula (I) tend to be unstable in aqueous solution over a period of time.

In addition to the aforementioned ingredients, the formulations of this invetnion may further include one or more accessory ingredient(s) selected from diluents, buffers, flavouring agents, binders, surface active agents, thickeners, lubricants, preservatives (including antioxidants) and the like.

Compounds of the formula (I) are particularly useful in increasing the synthesis of tyrosine, dopamine, norephinephrine and serotonin in mammals such as rats and humans. Such effects are produced by administration preferably orally or parenterally.

The following examples are provided by the way of illustration of the present invention and should in no way be construed as a limitation thereof.

Example 1

(+−)-2-Amino-5,6,7,8-tetrahydro-6-methoxymethyl-4(3H)-pteridinone

2-Amino-3-cyano-5-chloromethyl pyrazine (0.95 g) was reacted with a large excess of dry methanol, under nitrogen, at reflux, for about 18 hours. The resulting reaction mixture was filtered and the filtrate was taken to dryness by evaporation. A yellow residue was collected and extracted twice with boiling methylene chloride. These extracts were filtered and the solvent evaporated to give 2-amino-3-cyano-5-methoxymethylpyrazine which was then treated with an excess of guanidine in methanol according to the method of Taylor (*J. Org. Chem.,* 2817, *38,* (1973)). Most of the product of this reaction precipitated and was collected by filtration. The filtrate was mixed with silica gel, stripped of solvent by evaporation, and the residue was placed on a silica gel column. This column was eluted with methanol (5%) in ethylacetate to yield a second crop of product which was added to that previously collected. The combined crops were recrystallized from methanol to yield 2,4-diamino-6-methoxymethyl pteridine (mp 248—251°).

The diaminopteridine, *vide supra,* was hydrolyzed in 1N NaOH at 70°C for 3 hr. The resulting basic solution was then acidified with acetic acid, and the yellow product was filtered, washed with 95% ethanol and ethyl ether, and dried under vacuum overnight to yield 0.5 g (83%) of 2-amino-6-methoxymethyl-4(3H)pteridinone as a yellow powder: UV (0.1N NaOH)$\lambda_{max}$ 255 nm (22300), 277.5 nm sh (6200), 364 nm (7100); NMR (DMSO-$d_6$) δ 3.36 (s, OMe), 4.53 (s, $CH_2O$), 7.32 (brs, $NH_2$), and 8.63 (s, 7-H). Anal. calcd. for $C_8H_9N_5O_2$.7/1O$H_2O$: C, 43.72; H, 4.77; N, 31.86. Found: C, 43.77; H, 4.77; N, 31.63.

The pteridinone (0.5 g, $2.4 \times 10^{-3}$ mol) previously prepared, *vide supra,* was hydrogenated under 1.013 bar (1 atm) of $H_2$ in 30 ml of distilled trifluoroacetic acid over 0.02 g of $PtO_2$ for an hour. The mixture was diluted with 25 ml of 6N HCl, filtered, and evaporated. The crude product, the tetrahdyrocompound, was recrystallized twice from 6N HCl/acetonitrile to give 2-amino-5,6,7,8-tetrahydro-6-methoxymethyl-4(3H)-pteridinone as a light brown solid (0.6 g, 85%): UV (0.1 N HCl) $\lambda_{max}$ 215.5 nm (19800), 264 nm (19900); calcd. for $C_8H_{12}N_5O_2$ HCl. 1/2 $H_2O$: C, 32.78; H, 5.50; N, 23.89; Cl, 24.19. Found: C, 32.75; H, 5.54; N, 23.80; Cl, 24.21.

Example 2

(+−)-2-Amino-5,6,7,8-tetrahydro-6-n-butoxymethyl-4(3H)-pteridinone

This compound was made in a manner similar to that of the 6-methoxymethyl compound described in Example 1 (*vide supra*) except for the two following major exceptions: the n-butoxymethylpyrazine was made by heating the chloromethylpyrazine VI in n-butanol instead of methanol, and the guanidine reaction was carried out in heated n-butanol instead of refluxing methanol.

Spectra and microanalytical data of the three intermediate and final product are the following:

2-Amino-3-cyano-5-n-butoxymethylpyrazine

NMR ($Me_2SO-d_6$)δ 0.89 (t, 3H), 1.1—1.8 (m, 4H), 3.43 (t, 2H), 4.35 (s, 2H), 7.22 (brs, 2H), 8.25 (s, 1H); IR (CHCl3) 3694, 3526, 3414, 2930, 2866, 2220 (CN), 1723, 1612, 1463, 1377, 1275, 1088 $cm^{-1}$.

2,4-Diamino-6-n-butoxymethylpteridine

mp 237—238°C dec; NMR ($Me_2SO-d_6$) δ 0.87 (t, 3H), 1.0—1.7 (m, 4H), 3.05 (t, 2H), 4.58 (s, 2H), 6.63 (brs, 2H), 7.59 (brs, 2H), 8.73 (s, 1H). Anal. Calcd. for $C_{11}K_{16}N_6O$: C, 53.21; H, 6.50; N, 33.85. Found C, 53.02; H, 6.51; N, 33.75.

2-Amino-6-n-butoxymethyl-4(3H)-pteridinone

mp>300°C, UV (0.1 N NaOH) $\lambda_{max}$ 2.55.5 (26,900), 2755.5 sh (10,600), 364 (7,400) nm; NMR ($Me_2SO-d_6$) δ 0.87 (t, 3H), 1.0-1.8 (m, 4H), 3.50 (t, 2H), 4.57 (s, 2H), 6.97 (brs, 2H), 8.67 (s, 1H) (ring NH exchanged out due to large amount of $H_2O$ present); Anal. Calcd. for $C_{11}H_{15}N_5O_2$. 0.4 $H_2O$: C, 51.51; H, 6.21; N, 27.31. Found: C, 51.45; H, 6.13; N, 27.35.

(+−)-2-Amino-5,6,7,8-tetrahydro-6-n-butoxymethyl-4(3H)-pteridinone

mp 211—212°C dec; UV (0.1 N HCl) $\lambda_{max}$ 265 (15,800) nm; NMR ($Me_2SO-d_6$) δ 0.88 (t, 3H), 1.0—1.7 (m, 4H), 3.0—3.8 (m, 7H), 4.95, 6.85, 7.45, 10.5 (br bumps). Anal. Calcd. for $C_{11}H_{19}N_5O_2$. 1.75 HCl. $H_2O$: C, 39.42; H, 6.84; N, 20.90; Cl, 18.52. Found; C, 39.40; H, 6.74; N, 21.06; Cl, 18.61.

## Example 3

### 2-Amino-7,8-dihydro-6-methoxymethyl-7,7-dimethyl-4(3H) pteridinone dihydrochloride

A mixture of 2-amino-7,8-dihydro-6-methymethyl-7,7 dimethyl 4(3H) pteridinone, 0.10 g and platinum oxide (prereduced) 0.015 g, in 6 ml of 2N HCl was hydrogenated at 1.013 bar (1atm) $H_2$ at room temperature for 2.5 hr; the mixture was filtered, and the filtrate was evaporated. This crude product was recrystallized from 6N HCl/MeCN to yield 2-amino-5,6,7,8,-tetrahydro-6-methoxymethyl-7,7-dimethyl-4(3H)-pteridinone hydrochloride 0.089 g as a white solid, m.p. 214 300°C (dec), 76% of theoretical yield.

Elemental analysis: Calcd. for $C_{10}H_{17}N_5O_2$ 2HCl: C, 38.47; H, 6.13; N, 22.43; Cl, 22.71. Found: C, 38.33; H, 6.16; N, 22.34; Cl, 22.71.

## Examples 4 and 5

The following compounds were prepared from their corresponding precursors by the method described in example 3.

4. 2-Amino-5,6,7,8-tetrahydro-7-hydroxymethyl-6,7-dimethyl-4(3H)-pteridinone. m.p.>300°C.

Elemental analysis: Calcd. for $C_9H_{15}N_5O$.1/20 $C_9H_{13}N_5O$ 23/20 HCl. 11/25 $C_3H_8$ 0.3/20 $H_2O$: C, 42.37; H, 6.79; N, 22.85; Cl, 13.10. Found: C, 42.41; H, 6.75; N, 23.89; Cl, 13.10.

5. 2-Amino-5,6,7,8-tetrahydro-6-hydroxymethyl-7,7-dimethyl-4(3H)-pteridinone dihydrochloride. m.p. 200°C (dec).

Elemental analysis: Calcd. for $C_9H_{15}N_5O_2$.2HCl: C, 36.25; H, 5.75; N, 23.49; Cl, 23.78. Found: C, 36.13; H, 5.77; N, 23.42; Cl, 23.65.

## Example 6

### (+−)-2-Amino-6-butoxymethyl-5,6,7,8-tetrahydro-7,7-dimethyl-4(3H)-pteridinone dihydrochloride hemihydrate

A solution of 2-amino-6-butoxymethyl-7,8-dihydro-7,7-dimethyl-4(3H)-pteridinone (153 mg, 0.548 mmol) in trifluoroacetic acid (3ml) was hydrogenated over platinum oxide (11.3 mg) under 1.013 bar (1 atm) of $H_2$ at room temperature for 15 min. The product was worked up as before.

Yield: 0.15 g (75%) of white power: m.p. dec. above 200°C;

*IR* (KBr) 2960, 2870, 1650, 1570, 1450, 1400, 1180, 1120, 735 $cm^{-1}$; *UV* (0.1N HCl) $\lambda_{max}$: 217 nm (e) (19900), 263.5 (14300), λmin: 239 (4000); $^1H$—NMr (DMSO-$d_6$) δ from TMS: 0.88 (m, 3H, Me), 1.14 (S, 3H, Me), 1.28 (s, 3H, Me), 1.28 (s, 3H, Me), 1.44 (M, 4H, $CH_2CH_2$), 3.44 (M, 5H, C-6H, $CH_2CH_2$), rest exchanged.

Anal. Calc'd for $C_{13}H_{23}N_5O_2$ 2HCl $H_2O$:

C, 42.98; H, 7.21; N, 19.28; Cl, 19.52.

Observed: C, 42.95; H, 7.22; N, 19.48; Cl, 19.51.

Using a method analogous to that described in Example 6 the following compounds were prepared.

## Example 7

### (+−)-2-Amino-5,6,7,8-tetrahydro-6-idsopentyloxymethyl-7,7-dimethyl-4(3H)-pteridinone dihydrochloride

M.p. dec. above 190°C.

Anal. calc'd for $C_{14}H_{25}N_5O_2$ HCl:

C, 45.65; H, 7.39; N, 19.02; Cl, 19.25.

Observed: C, 45.53; H, 7.40; N, 19.00; Cl, 19.11.

Example 8

(+−)-2-Amino-5,6,7,8-tetrahydro-7,7-dimethyl-6-octyloxymethyl-4(3H)-pteridinone dihydrochloride
M.p. dec. above 200°C.
Anal. calc'd for $C_{17}H_{21}N_5O_2$ 2 HCl:
C, 49.75; H, 8.10; N, 17.07; Cl, 17,28.
Observed: C, 49.68; H, 8.13; N, 17.07; Cl, 17.26.

Example 9

(+,−)-2-Amino-5,6,7,8-tetrahydro-7,7-dimethyl-6-pentoxymethyl-4(3H)-pteridinone dihydrochloride
M.p. dec. above 190°C.
Anal. calc'd for $C_{14}H_{25}N_5O_2$ 2 HCl:
C, 45.65; H, 7.39; N, 19.02; Cl, 19.25.
Observed: C, 45.63; H, 7.43; N, 19.02; Cl, 19.19.

Example 10

(+,−)-2-Amino-6-(3-cyclohexylpropoxymethyl)-5,6,7,8-tetrahydro-7,7-dimethyl-4(3H)-pteridinone dihydrochloride
M.p. 216—221°C (dec).
Anal. calc'd for $C_{18}H_{31}N_5O_2$ 2 HCl:
C, 51.18; H, 7.87; N, 16.58; Cl, 16.79.
Observed: C, 50.97; H, 7.91; N, 16.52; Cl, 16.33.

Example 11

(+,−)-2-Amino-5,6,7,8-tetrahydro-7,7-dimethyl-6-(3-phenylpropoxymethyl)-4(3H)-pteridinone dihydrochloride
M.p. 194—200°C (dec).
Anal. calc'd for $C_{18}H_{25}N_5O_2$ 2HCl:
C, 51.92; H, 6.54; N, 16.82; Cl, 17.03.
Observed: C, 51.73; H, 6.57; N, 16.72; Cl, 17.03.

Example 12

(+,−)-2-Amino-5,6,7,8-tetrahydro-6-isopropoxymethyl-7,7-dimethyl-4(3H)-pteridinone dihydrochloride-one-quarter hydrate
M.p. 190—192°C (dec).
Anal. calc'd for $C_{12}H_{21}N_5O_2$ 2HCl 1/4 $H_2O$:
C, 41.81; H, 6.87; N, 20.31; Cl, 20.57.
Observed: C, 41.79; H, 6.89; N, 20.27; Cl, 20.59.

Example 13

(+,−)-2-Amino-5,6,7,8-tetrahydro-7,7-dimethyl-6-propoxymethyl-4(3H)-pteridinone dihydrochloride
Anal. calc'd for $C_{12}H_{21}N_5O_2$ 2HCl:
C, 42.36; H, 6.31; N, 20.58.
Observed: C, 42.31; H, 6.81; N, 20.53.

Example 14

(+,−)-2-Amino-5,6,7,8-tetrahydro-7,7-dimethyl-6-propyl-4(3H)-pteridinone dihydrochloride
UV (0.1N HCl)$\lambda_{max}$: 263.5, 214 nm; λmin: 238.5 nm.

Example 15

(+,−)-2-Amino-5,6,7,8-tetrahydro-6-isopropyl-7,7-dimethyl-4(3H)-pteridinone dihydrochloride
UV (0.1N HCl)$\lambda_{max}$: 264, 217 nm; λmin: 239 nm.

Example 16

(+,−)-2-Amino-6-ethyl-5,6,7,8-tetrahydro-7,7-dimethyl-4(3H)-pteridinone dihydrochloride hemihydrate
Anal. Calc'd for $C_{10}H_{17}N_5O$ 2 HCl 1/2 $H_2O$:
C, 39.35; H, 6.60; N, 22.95; Cl, 23.23.
Observed: C, 39.11; H, 6.51; N, 22.95; Cl, 23.26.

Example 17

(+,−)-2-Amino-6-ethoxymethyl-5,6,7,8-tetrahydro-7,7-dimethyl-4(3H)-pteridinone dihydrochloride 3/4 hydrate
M.p. = 203—207°C (dec).
Anal. Calc'd for $C_{11}H_{19}N_5O_2$ 2 HCl 3/4 $H_2O$:
C, 38.89; H, 6.68; N, 20.61; 20.87.
Observed: C, 38.92; H, 6.64; N, 20.72; Cl, 20.77.

Example 18
Biological Data

Tyrosine hydroxylate was partially purified through a 25—45% ammonium sulfate fraction. Specific activity of this preparation with tetrahydrobiopterin was 4.0 nmole/mg protein (min). the enzyme was assayed by a modification of the method of Nagatsu, T., Levitt, M., and Udenfriend S. (*Anal. Biochem. 9,* 122, (1964)) where the Dowex chromatography step was replaced by a charcoal extraction.

Tryptophan hydroxylate was assayed according to a modification of the method of Renson J., *et al* (*Biochem. Biophys. Acta 25*: 504 (1966)). The preparation of tryptophan hydroxylase was a crude 30,000xg supernatant which had been desalted on a Sephadex G-25 (Trade Name) column (Boadle-Biker, M.C., *Biochem Pharmacol. 28*: 2129 (1979). The specific activity of this tryptophan hydroxylase, using $BH_4$ as cofactor, was approximately 100 pmol product per milligram protein per minute at 37°.

Phenylalanine hydroxylase was measured as described by Shiman, R. *et al.,* (*J. Biol. Chem. 254*: 11300 (1979)) except that the product of the reaction (tyrosine) was measured fluorometrically using the method of Waalkes, T.P. and Udenfriend, S. [*J. Lab. Clin. Med. 50*: 733 (1957). Phenylalanine hydroxylase was prepared from rat liver using hydrophobic chromatography (Shiman, R. *et al.* (*J. Biol. Chem. 254*: 11,300 (1979)). The specific activity of the enzyme, using $BH_4$ as the co-factor, was 1.0 μmol/mg protein per minute at 37°. Dihydropteridine reduction was assayed by the method of Crane, *et al.* (*J. Biol. Chem,, 247,* 6082 (1972)).

TABLE 1

Biological Activity of Various Pterins

| Compound of Formula (I) $R^1$ | $R^2$ | $R^3$ | $R^4$ | $V_{max}$(%) Tyrosine Hydroxylase | Tryptophan Hydroxylase | Phenylalanine Hydroxylase | Dihydropteridine reductase |
|---|---|---|---|---|---|---|---|
| Tetrahydrobiopterin ($BH_4$) | | | | 100 | 100 | 100 | 100 |
| $CH_2$—O —$CH_3$ | H, | $CH_3$ | $CH_3$ | 138 | 0 | 5 | 316 |
| $CH_2OH$, | H, | $CH_3$ | $CH_3$ | 102 | 0 | 5 | 161 |
| $CH_3$, | H, | $CH_3$ | $CH_3$ | 54 | 0 | 43 | 316 |
| $CH_3$, | H, | $CH_3$ | $CH_2OH$ | 25 | 0 | 3 | 81 |
| H, | H, | $CH_3$ | $CH_3$ | 83 | 0 | 5 | 277 |
| $CH_2OCH_3$ | H, | H | H | 124 | 54 | 79 | 295 |

Numbers are percent of maximal velocities, obtained with $BH_4$. Enzyme assays were performed as described in the text.

Example 19
Pharmaceutical Formulations

A. Capsule

| Ingredient | Amount per capsule (mg) |
|---|---|
| Compound I | 325.0 |
| Ascorbic Acid | 174.0 |
| Corn Starch | 100 mg |
| Stearic Acid | 27 mg |

The finely ground active compound was mixed with the powdered excipients lactose, corn starch and stearic acid and packed into a gelatin capsule.

B. Tablet

| Ingredient | Amount per tablet (mg) |
|---|---|
| Compound I | 325.0 |
| Ascorbic Acid | 125.0 |
| Corn Starch | 50.0 |
| Polyvinylpyrrolidone | 3.0 |
| Stearic Acid | 1.0 |
| Magnesium stearate | 1.0 |

The active compound was finely ground and intimately mixed with the powdered excipients lactose, corn starch, polyvinylpyrrolidone, magnesium stearate and stearic acid. The formulation was then compressed to afford one tablet weighing 505 mg.

A. Suppository

| Ingredient | Amount per suppository |
|---|---|
| Compound I | 325.0 mg |
| Butylate hydroxy toluene (BHT) | 25 mg |
| Cocoa Butter or Wecobee* Base q.s | 2.0 g |

* Wecobee is the trade name of a hydrogenated carboxylic acid.

Scheme I

Representative synthetic Routes to Compounds of the Formula IV.

1).

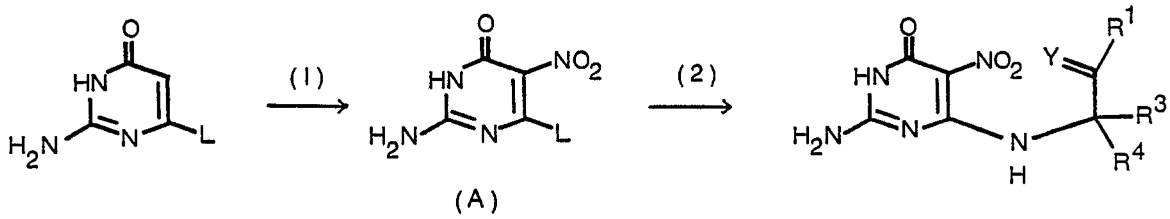


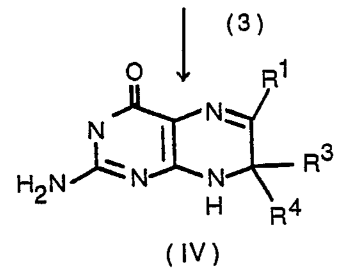


L = Leaving Group

Y = O, NOR, S

1) $-HNO_3$, $H_2SO_4$

2) –

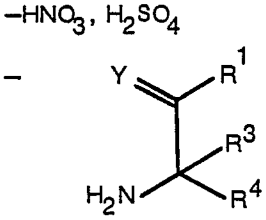

3) [H]

2).

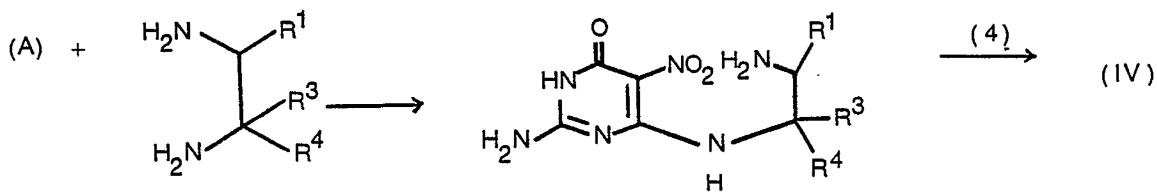


(4) heat or [H] followed by $O_2$

3).

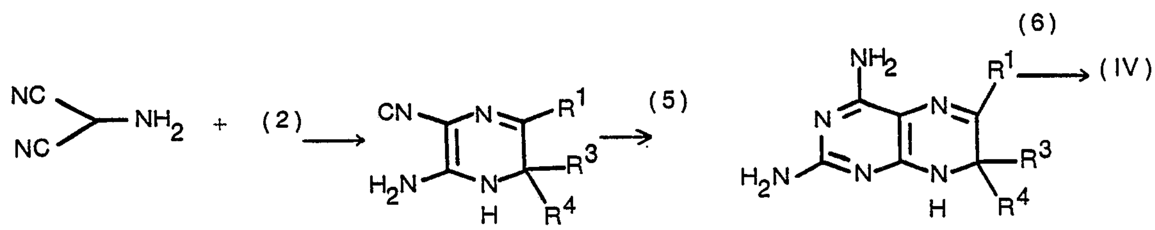


(5) – guanidine

(6) – NaOH

4).

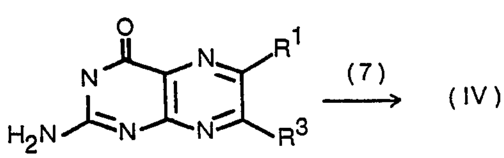


(7) – [H]

5). 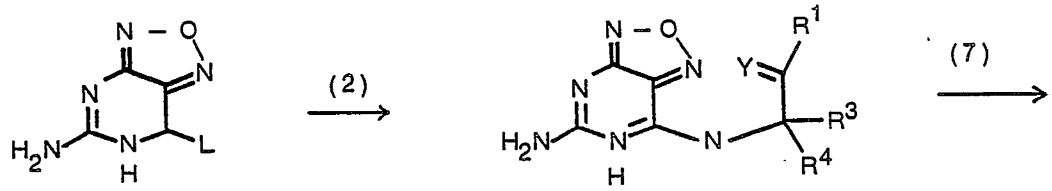

(IV)

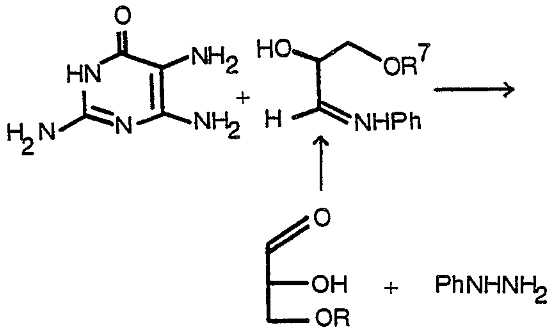
(IV)

## Claims

1. A compound of the formula (I):

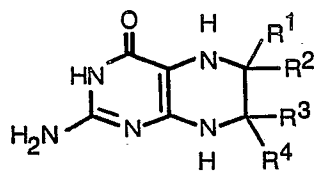
(I)

or a pharmaceutically acceptable salt thereof; wherein $R^1$ is hydrogen, $C_{1-4}$ alkyl, $C_{2-4}$ alkenyl, a group $—BX(R^5)n$, or $—BZBX—(R^5)n$ wherein Z and X (when n = 1) are the same or different and each is —O—, $—S(O)_q—$ or $—NR^6—$ wherein q is 0, 1 or 2 and $R^6$ is hydrogen or $C_{1-4}$ alkyl or, when n = 0, X is halogen; $R^5$ is hydrogen, up to $C_{12}$ aralkyl, up to $C_{10}$ alkyl or cycloalkyl; B is $C_{1-5}$ alkylene; $R^2$ is hydrogen $C_{1-4}$ alkyl, $C_{2-4}$ alkenyl or a group $BOR^7$ wherein B is as hereinbefore defined and $R^7$ is hydrogen or $C_{1-6}$ alkyl; or $R^1$ and $R^2$ together with the carbon atom in the pteridine ring structure to which they are attached, form a $C_{3-7}$ spirocycloalkyl ring; $R^3$ and $R^4$ are hydrogen or methyl; or $R^2$ and $R^3$ together with the carbon atoms in the pteridine ring structure to which they are attached form a $C_{5-7}$ cycloalkyl ring; or a bioprecursor thereof according to formula (IV) or (V)

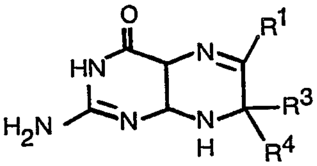
(IV)

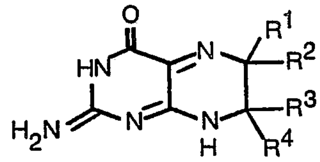
(V)

wherein $R^1$, $R^2$, $R^3$, $R^4$ are as hereinbefore defined with the proviso that when $R^1$ in formula (IV) is methyl, hydroxymethyl or bromomethyl, then $R^3$ and $R^4$ are not both methyl; and the compounds 2-amino-4-hydroxy-6-hydroxymethyl-7,7-dimethyl-7,8-dihydropteridine; 2-amino-4-hydroxy-6-methyl-7,7-dimethyl-7,8-dihydropteridine; 2-amino-4-hydroxy-6-bromomethyl-7,7-dimethyl-7,8-dihydropteridine being excluded.

2. A compound of formula (II)

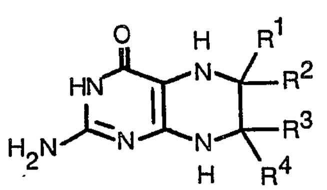
(II)

or a pharmaceutically acceptable salt or bioprecursor thereof as defined in claim 1; wherein $R^1$ to $R^4$ are as defined in claim 1 and at least one of $R^1$, $R^2$ and $R^3$, $R^4$ represents gem disubstitution.

3. A compound of formula (III)

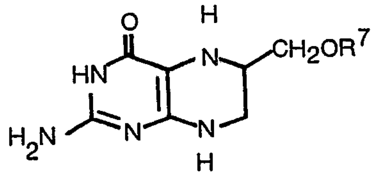

(III)

or a pharmaceutically acceptable salt or bioprecursor thereof as defined in claim 1; wherein $R^7$ is as defined in claim 1.

4. A compound of formula (I) or a pharmaceutically acceptable salt thereof as defined in claim 1, provided that when $R^3$ and $R^4$ are methyl, neither $R^1$ nor $R^2$ are methyl.

5. A compound of formula (II) or a pharmaceutically acceptable salt thereof as defined in claim 2, provided that when $R^3$ and $R^4$ are methyl, neither $R^1$ nor $R^2$ are methyl.

6. A compound of formula (III) or a pharmaceutically acceptable salt thereof as defined in claim 3 or bioprecursor thereof as defined in claim 1.

7. A compound according to claim 4 or 5 wherein B is $CH_2$, X is oxygen and $(R^5)n$ represents hydrogen or a $C_{1-10}$ alkyl group.

8. A pharmaceutical composition which comprises a compound of formula (I) or a pharmaceutically acceptable salt thereof or a bioprecursor thereof as defined in claim 1, together with one or more pharmaceutically acceptable carriers.

9. A pharmaceutical composition according to claim 8 which contains other active therapeutic ingredients.

**Patentansprüche**

1. Verbindung der Formel (I):

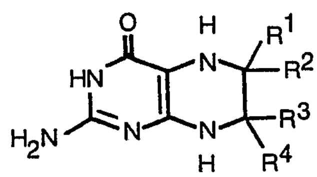

(I)

oder ein pharmazeutisch annehmbares Salz davon, worin $R^1$ Wasserstoff, $C_{1-4}$ Alkyl, $C_{2-4}$ Alkenyl, eine Gruppe —$BX(R^5)n$ oder —$BZBX(R^5)n$ ist, worin Z und X (wenn n = 1) gleich oder verschieden sind und jeweils —O—, —S(O)q— oder —$NR^6$— bedeuten, worin q 0, 1 oder 2 ist und $R^6$ Wasserstoff oder $C_{1-4}$ Alkyl ist, oder, wenn n = 0 ist X Halogen, $R^5$ Wasserstoff, bis zu $C_{12}$ Aralkyl, bis zu $C_{10}$ Alkyl oder Cycloalkyl; B ist $C_{1-5}$ Alkylen; $R^2$ ist Wasserstoff, $C_{1-4}$ Alkyl, $C_{2-4}$ Alkenyl oder eine Gruppe $BOR^7$, worin B die oben gegebene Bedeutung besitzt und $R^7$ Wasserstoff oder $C_{1-6}$ Alkyl ist; oder $R^1$ und $R^2$ bilden zusammen mit den Kohlenstoffatomen in der Pteridinringstruktur, an die sie gebunden sind, einen $C_{3-7}$ Spirocycloalkylring; $R^3$ und $R^4$ sind Wasserstoff oder Methyl; oder $R^2$ und $R^3$ bilden zusammen mit den Kohlenstoffatomen in der Pteridinringstruktur, an die sie gebunden sind, einen $C_{5-7}$ Cycloalkylring; oder eine Biovorstufe davon gemäß Formel (IV) der (V):

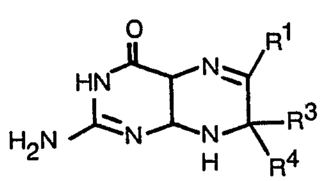

(IV)

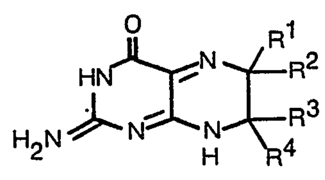

(V)

worin $R^1$, $R^2$, $R^3$, $R^4$ die oben gegebene Bedeutung bezitzen, mit der Maßgabe, daß wenn $R^1$ in Formel (IV) Methyl, Hydroxymethyl oder Brommethyl ist, dann sind $R^3$ und $R^4$ nicht beide Methyl; und wobei die Verbindungen 2-Amino-4-hydroxy-6-hydroxymethyl-7,7-dimethyl-7,8-dihydropteridin; 2-Amino-4-hydroxy-6-methyl-7,7-dimethyl-7,8-dihydropteridin; 2-Amino-4-hydroxy-6-brommethyl-7,7-dimethyl-7,8-dihydropteridin ausgeschlossen sind.

2. Verbindung der Formel (II)

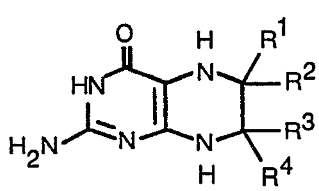

(II)

oder ein pharmazeutisch annehmbares Salz davon oder eine Biovorstufe davon wie in Anspruch 1 definiert, worin $R^1$ bis $R^4$ die in Anspruch 1 gegebenen Bedeutungen bezitzen und mindestens eines von $R^1$, $R^2$ und $R^3$, $R^4$ eine Gem-Disubstitution aufweist.

3. Verbindung der Formel (III):

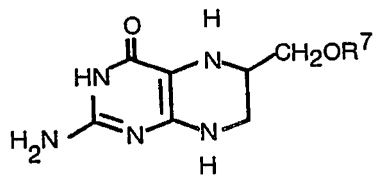

(III)

oder ein pharmazeutisch annehmbares Salz oder eine Biovorstufe davon, wie in Anspruch 1 definiert, worin $R^7$ die in Anspruch 1 gegebene Bedeutung besitzt.

4. Verbindung der Formel (I) oder ein pharmazeutisch annehmbares Salz davon, wie in Anspruch 1 definiert, mit der Maßgabe, daß, wenn $R^3$ und $R^4$ Methyl sind, weder $R^1$ noch $R^2$ Methyl ist.

5. Verbindung der Formel (III) oder ein pharmazeutisch annehmbares Salz davon, wie in Anspruch 2 definiert, mit der Maßgabe, daß wenn $R^3$ und $R^4$ Methyl sind, weder $R^1$ noch $R^2$ Methyl sind.

6. Verbindung der Formel (III) oder ein pharmazeutisch annehmbares Salz davon, wie in Anspruch 3 definiert, oder eine Biovorstufe davon, wie in Anspruch 1 definiert.

7. Verbindung nach Anspruch 4 oder 5, worin B $CH_2$, X Sauerstoff ist und $(R^5)n$ Wasserstoff oder eine $C_{1-10}$ Alkylgruppe bedeutet.

8. Pharmazeutisch Zusammensetzung, die eine Verbindung der Formel (I) oder ein pharmazeutisch annehmbares Salz davon oder eine Biovorstufe davon, wie in Anspruch 1 definiert, zusammen mit einem oder mehreren pharmazeutisch annehmbares Trägerstoffen enthält.

9. Pharmazeutisch Zusammensetzung nach Anspruch 8, die andere therapeutisch aktive Bestandteile enthält.

**Revendications**

1. Composé de formule (I):

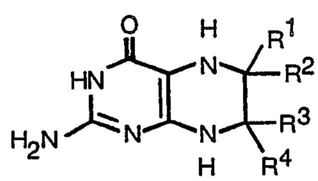

(I)

ou un sel pharmaceutiquement acceptable de celui-ci; où $R^1$ est un atome d'hydrogène, un radical $C_{1-4}$ alcoyle ou $C_{2-4}$ alcényle ou un radical $—BX(R^5)n$ ou $—BZBX(R^5)n$ où Z et X (lorsque n = 1) sont identiques ou différents et sont chacun —O—, —S(O)q— ou $—NR^6—$, où q est 0, 1 ou 2 et $R^6$ est un atome d'hydrogène ou radical $C_{1-4}$-alcoyle ou bien lorsque n = 0, X est un atome d'halogène; $R^5$ est un atome d'hydrogène ou radical aralcoyle jusqu'en $C_{12}$, ou alcoyle ou cycloalcoyle jusqu'en $C_{10}$; B est un radical $C_{1-5}$ alcoylène; $R^2$ est un atome d'hydrogène, un radical $C_{1-4}$ alcoyle ou $C_{2-4}$ alcényle ou un radical $—BOR^7$ où B est tel que défini ci-dessus et $R^7$ est un atome d'hydrogène ou radical $C_{1-6}$ alcoyle; ou bien $R^1$ et $R^2$ conjointement avec l'atome de carbone de la structure cyclique de ptéridine auquel ils sont unis forment un cycle $C_{3-7}$ spiro-cycloalcoyle; $R^3$ et $R^4$ sont des atomes d'hydrogène ou radicaux méthyle; ou bien $R^2$ et $R^3$ conjointement avec les atomes de carbone de la structure cyclique de ptéridine auxquels ils sont unis forment un radical $C_{5-7}$ cycloalcoyle; ou un bioprécurseur correspondant de formule (IV) ou (V)

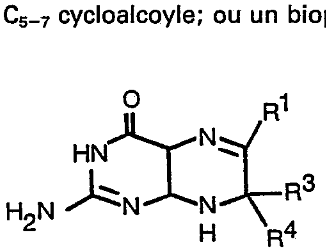

(IV)

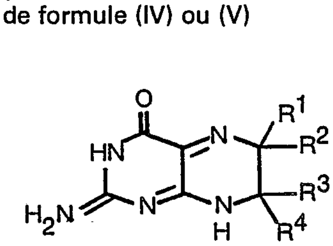

(V)

où $R^1$, $R^2$, $R^3$ et $R^4$ sont tels que définis ci-dessus, avec la restriction que lorsque $R^1$ dans la formule (IV) est un radical méthyle, hydroxyméthyle ou bromométhyle, $R^3$ et $R^4$ ne sont alors pas tous deux des radicaux méthyle, et que la 2-amino-4-hydroxy-6-hydroxyméthyl-7,7-diméthyl-7,8-dihydroptéridine, la 2-amino-4-hydroxy-6-méthyl-7,7-diméthyl-7,8-dihydroptéridine et la 2-amino-4-hydroxy-6-bromométhyl-7,7-diméthyl-7,8-dihydroptéridine sont exclues.

2. Composé de formule (II):

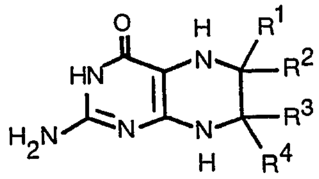

(II)

ou un sel pharmaceutiquement acceptable ou bioprécurseur de celui-ci tel que défini dans la revendication 1, où $R^1$ à $R^4$ sont tels que définis dans la revendication 1 et au moins l'un d'entre $R^1$, $R^2$ et $R^3$, $R^4$ représente une gem-disubstitution.

3. Composé de formule (III):

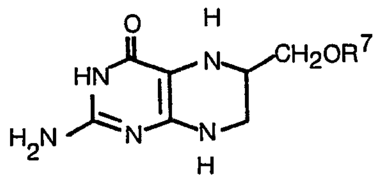

(III)

ou un sel pharmaceutiquement acceptable ou bioprécurseur de celui-ci tel que défini dans la revendication 1, où $R^7$ est tel que défini dans la revendication 1.

4. Composé de formule (I) ou un sel pharmaceutiquement acceptable de celui-ci tel que défini dans la revendication 1, avec la restriction que lorsque $R^3$ et $R^4$ sont des radicaux méthyle, ni $R^1$, ni $R^2$ ne sont des radicaux méthyle.

5. Composé de formule (II) ou un sel pharmaceutiquement acceptable de celui-ci tel que défini dans la revendication 2, avec la restriction que lorsque $R^3$ et $R^4$ sont des radicaux méthyle, ni $R^1$, ni $R^2$ ne sont des radicaux méthyle.

6. Composé de formule (III) ou un sel pharmaceutiquement acceptable de celui-ci tel que défini dans la revendication 3 ou un bioprécurseur de celui-ci tel que défini dans la revendication 1.

7. Composé suivant la revendication 4 ou 5 où B est un radical $CH_2$, X est un atome d'oxygène et $(R^5)n$ représente un atome d'hydrogène ou radical $C_{1-10}$ alcoyle.

8. Composition pharmaceutique, qui comprend un composé de formule (I) ou un sel pharmaceutiquement acceptable de celui-ci ou bien un bioprécurseur de celui-ci tels que définis dans la revendication 1, conjointement avec un ou plusieurs excipients pharmaceutiquement acceptables.

9. Composition pharmaceutiquement suivant la revendication 8, qui contient d'autres constituants thérapeutiquement actifs.